# EUROPEAN PATENT APPLICATION

(11) **EP 3 290 065 A1**
(43) Date of publication of application: **07.03.2018**
(21) Application number: 17188405.9
(22) Date of filing: 29.08.2017
(51) Int. Cl.: A61M 1/02, A61M 1/34

(54) **EXTRACTOR APPARATUS FOR BLOOD COMPONENTS**

(30) Priority: 29.08.2016 IT 201600087910
(71) Applicant: Delcon S.r.l., 20131 Milano (IT)
(72) Inventor: Sala, Norberto, 20862 Arcore (MB) (IT)
(74) Representative: Petraz, Gilberto Luigi

(57) **Abstract**

Extractor apparatus for blood components, comprising a plurality of blood bags housings equipped with RFID communication modules, and multifunction clamps (14a, 14b, 14c, 14d, 14e) able to hold, control the flow of hematic fluid and weld at least one tube (60) allowing the transfer of at least one blood component between mother and satellite bags.

## Description

### FIELD OF THE INVENTION

The present invention concerns an apparatus for extracting blood components, which can be used, in particular, in the medical field of blood transfusion, to extract, from a mother-bag containing centrifuged whole blood, blood components such as red blood cells, platelets and plasma that are collected in corresponding satellite bags.

### BACKGROUND OF THE INVENTION

It is known that in the medical field of blood transfusion, the blood or blood fractions (blood derivatives and/or blood components), or hematic fluids in general, can be taken from and/or respectively given to the patient by the use of bags, normally containing an anticoagulant, connected to plastic tubes inside which the blood or blood fractions flow.

For example, in the case of blood donation, the donor is made to lie down on a bed, a tourniquet is attached to one arm and a needle is inserted into a vein. The needle is connected to a tube, in turn connected to a blood collection bag. The blood flows through gravity and venous pressure until it fills the collection bag which already contains an anticoagulant solution or liquid and other substances or solutions useful for optimum storage of the blood. Before the needle is extracted, some test tubes are filled to carry out the tests envisaged by the law. The volume of blood taken is generally established by law to ensure adequate preparation of the blood components (concentrates of red blood cells, platelets and plasma), and also to ensure absence of complications for the donor.

The blood or hematic fluid can be used as whole blood or as blood divided into its blood components, in which case the blood is subjected to a centrifugation process so as to obtain a stratification of the main blood components in the mother bag.

The main components are: red blood cells, which represent the densest layer, positioned on the bottom of the mother bag; platelets and white blood cells, which represent an intermediate layer, called "buffy coat"; and plasma, which represents the upper and least dense layer of the hematic fluid or centrifuged blood.

For the purpose of dividing the various components into satellite bags belonging to collection and processing kits, extractor apparatuses are known, provided with suitable housings for the mother bag containing centrifuged blood and for the satellite bags of the kit.

In such housings, the mother bag is subjected to a controlled pressing process so as to maintain the intermediate layer of platelets and white blood cells inside a certain level range, so that it does not rise or fall too much inside the mother bag, or in order to perform other controlled procedures of blood component extraction.

As is known, these apparatuses, for example in the case of so-called "top and bottom" bags, are provided with extraction means and valves which substantially allow the red blood cells to be extracted from the bottom of the bag and the plasma from the upper part of the bag, until the buffy coat intermediate layer remains in the mother bag with the desired quantity of plasma or percentage of red blood cells (hematocrit level).

These apparatuses are also provided with clamps to retain and weld the tubes that establish the hematic fluid pathways from the original bag of centrifuged blood to the satellite collection bags, and are also provided with a display associated with a series of keys by means of which to set the various removal functions and the desired modes of extracting the blood components.

However, these multifunction clamps are deficient in terms of controlling the flow of hematic fluid.

Another disadvantage of known apparatuses is due, for example, to the lack of effective elements which, according to the selected extraction program, indicate the need to insert a tube in a given clamp, and which also indicate the correct positioning of the tube inside the selected retention and welding clamp.

Further disadvantages related to known apparatuses and above all to the clamps also concern the difficulty of maintaining and cleaning them, especially inside the body supporting said clamps.

In these clamps, welding is normally done by radio-frequency systems provided with a pair of welding electrodes. Another disadvantage of known apparatuses is that both the welding electrodes, that is, the electrode receiving the radiofrequency signal and the ground electrode, are both attached to the clamp support body, and this positioning not only generates additional difficulties in maintaining and cleaning the clamp, but can also cause undesirable electrical instability in the clamp.

Known clamps also have sealing problems against possible fluid infiltration, particularly of hematic fluid, which can occur during the operations to extract blood components carried out by the apparatus, for example in the case of defects in the tubes, incorrect welding processes, erroneous handling operations or others.

Other limitations of known apparatuses concern, for example, the absence of means to automatically recognize the original bags and/or collection bags when they are positioned on the apparatus, limits in the controlled pressing operations on the original bag, or mother bag, lack of automatic congruence checks with transfusion center database, automatic launch of pre-defined procedures without operator intervention and limits from the general point of view of the efficiency and optimization of their use. Known apparatuses also do not include means for tracing the bags during the extraction process, or means for writing the result of the process on the bags themselves.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to obtain an apparatus for extracting blood components which can overcome at least one of the disadvantages of the state of the art.

One purpose of the present invention is to obtain an extractor apparatus which permits to bag recognition and check, automatic procedures and enhance traceability of the process with no need of operator intervention. One purpose of the present invention is to obtain an extractor apparatus for blood components which allows precise and rapid operations to extract the blood components from the mother bag.

One purpose of the present invention is therefore to obtain an extractor apparatus for blood components that is provided with multifunction clamps able to effectively and quickly indicate where to position the tubes through which the hematic fluid passes, and also to indicate the correct positioning of said tubes, so as to obtain an effective and optimal process for separating whole blood into its main blood components.

Another purpose of the present invention is to obtain an extractor apparatus for blood components which is provided with a series of multifunction clamps which guarantee electrical stability, which can be subjected effectively to cleaning and maintenance operations, even internally, and which guarantee a suitable seal against possible unwanted infiltrations of fluids.

Another purpose of the present invention is to obtain an extractor apparatus for blood components that is provided with means to automatically recognize the bags containing the hematic fluid, hence the mother bag and/or satellite bags.

Another purpose of the present invention is to obtain an extractor apparatus for blood components that has improvements in the means and operations for the controlled pressing of the bag, and that has improved optical elements to detect the intermediate level in the bag.

The Applicant has devised, tested and embodied the present invention to overcome the shortcomings of the state of the art and to obtain these and other purposes and advantages.

### SUMMARY OF THE INVENTION

The present invention is set forth and characterized in the independent claim, while the dependent claims describe other characteristics of the invention or variants to the main inventive idea.

In accordance with the above purposes, and according to a first aspect of the invention, an extractor apparatus for blood components comprises a plurality of multifunction clamps able to hold, control the flow of hematic fluid and weld at least one tube suitable for use in the field of medical blood transfusion and configured to connect a mother bag containing centrifuged whole blood to at least one satellite bag, thus allowing the transfer of at least one blood component between said bags.

According to another aspect of the invention, the apparatus comprises at least one housing seating of the mother bag and one or more housing seatings of said one or more satellite bags, said housing seating of the mother bag being provided with means for the controlled pressing of the mother bag.

According to another aspect of the invention, the multifunction clamps are provided with one or more optical elements able to indicate automatically and selectively at least a situation of programmed insertion of the tube and at least a situation of correct holding of the tube.

The multifunction clamps preferably comprise at least a sealing element against possible infiltrations of fluids.

According to some embodiments, the multifunction clamps comprise at least a support body with which a removable lid is associated.

The multifunction clamps can comprise at least a hot welding electrode and at least a cold welding electrode, at least one of either said hot and cold electrodes being associated with said lid.

The lid can be provided with at least a diffuser able to spread the light signals coming from said optical elements situated in a zone of the multifunction clamp below the lid.

Preferably, the hot electrode is associated with a mobile module provided with said sealing element.

In some embodiments, the multifunction clamps comprise at least one pair of uprights comprising grooves to hold the tube and one or more sensors to detect the presence of the tube.

According to another aspect of the invention, the extractor apparatus comprises one or more communication modules of the RFID type installable in correspondence to the housing seatings of the mother bag and/or the satellite bags, able to perform an automatic recognition of the RFID tag equipped mother bag and/or the satellite bags and to write data on the bags RFID tag.

On the bags obtained at the end of the extraction process, the data resulting from the extraction process will also be written, for example the weight of the component obtained, the extraction time and others.

In some embodiments, the extractor apparatus comprises a door to close the housing seating of the mother bag provided with semi-automatic opening means.

The pressing means can comprise at least a plate that has a substantially flat and uniform pressing surface.

In some embodiments, the housing seating of the mother bag comprises at least a column of optical elements of which at least one group is provided with one or more optical elements configured to detect precisely a range of the level of the layer of platelets and white blood cells to keep inside the mother bag.

These and other aspects, characteristics and advantages of the present disclosure will be better understood with reference to the following description, drawings and attached claims. The drawings, which are integrated and form part of the present description, show some embodiments of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a first three-dimensional view of an extractor apparatus for blood components according to the invention;
- fig. 2 is a second three-dimensional view of the present apparatus;
- fig. 3 is a first three-dimensional view of a multifunction clamp;
- fig. 4 is a second three-dimensional view of the multifunction clamp in fig. 3 provided with a lid;
- fig. 5 is a first longitudinal section view of the multifunction clamp in fig. 4 with the lid screwed on;
- fig. 6 is a second longitudinal section view, considered on a plane orthogonal to the longitudinal section plane of fig. 5.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

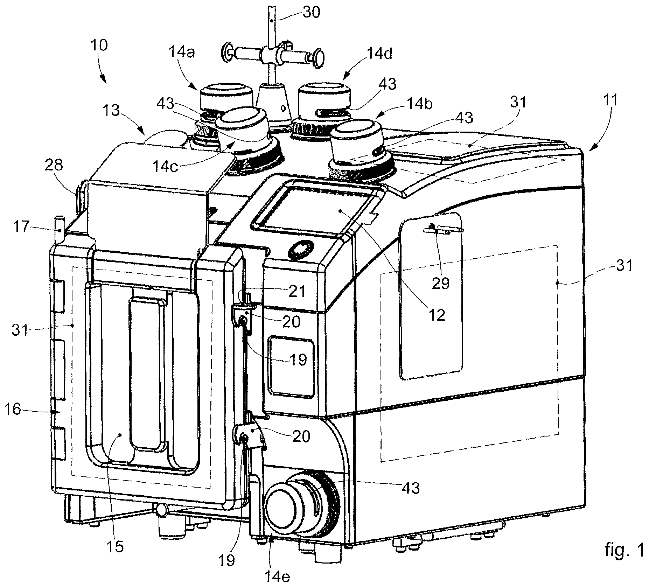

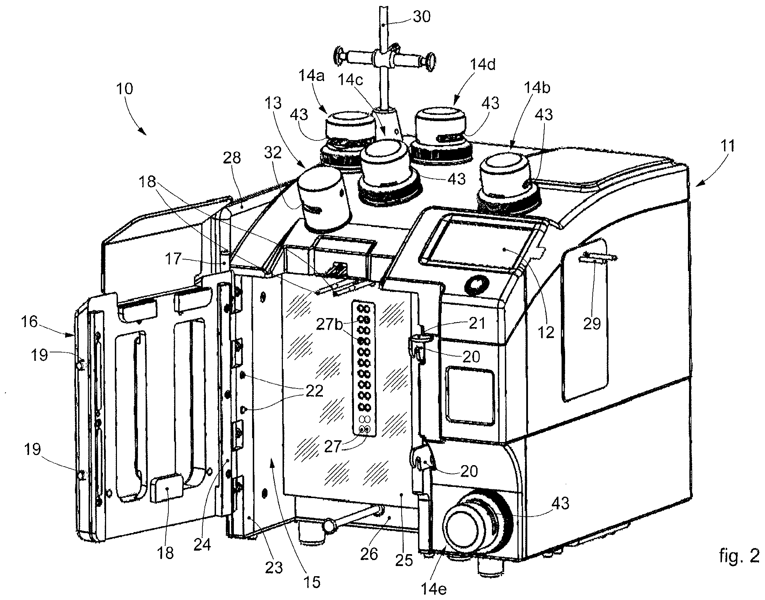

With reference to the attached drawings, and in particular to figs. 1 and 2, an apparatus 10 for extracting blood components comprises a box-like body 11 provided with a monitor 12, for example a touchscreen, for setting the selected extraction program and for displaying the extraction data.

The apparatus 10 comprises a first consent clamp 13 in which a tube is inserted, to be used in the field of blood transfusion and in particular for the passage of centrifuged whole blood arriving from a mother bag, not shown in the drawings and known per se.

The clamp 13 supplies information about the correct positioning of the tube and the presence of a flow of fluid inside the tube. In this regard, the clamp 13 comprises a housing seating 32 for housing the tube arriving from the mother bag.

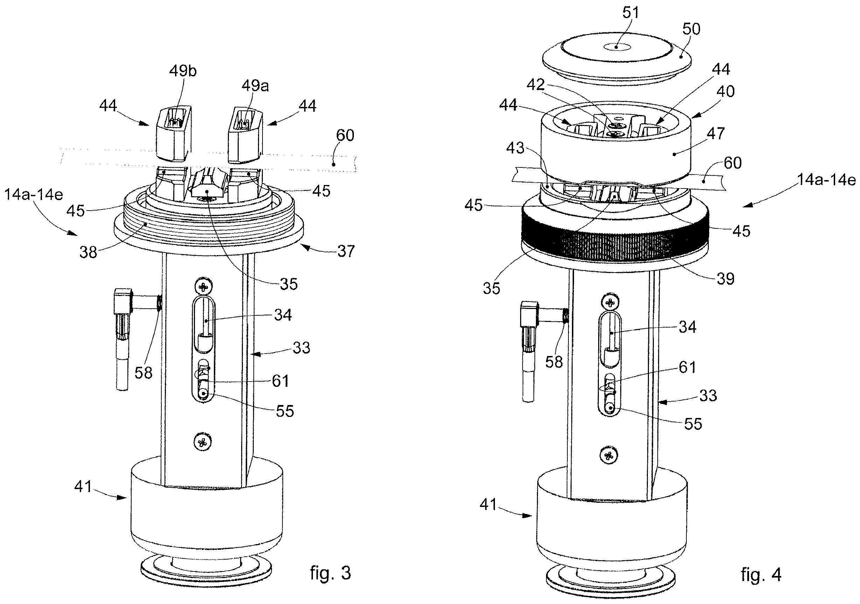

The apparatus comprises a plurality of multifunction clamps 14a, 14b, 14c, 14d and 14e in which a tube 60 to be used in the blood transfusion field and in particular for the passage of blood components, see figs. 3 to 6, can be selectively positioned.

The multifunction clamps 14a-14e are selectively used according to the selected extraction program.

The multifunction clamps 14a-14e, which will be described in detail hereafter, have mainly the function of retaining the tube in position, controlling the flow, and actuating a final welding of the tube 60 once the process of extracting the blood components is terminated. The flow control can be the open/close type or can provide a proportional regulation, for example in the multifunction clamp 14c.

The mother bag containing the centrifuged whole blood can be positioned in a seating 15 situated on the front side of the apparatus 10.

The seating 15, in which the mother bag is housed in a substantially vertical position, is delimited at the front by a door 16.

The door 16 is hinged on at least one lateral pin 17 which allows a certain rotation thereof in opening and closing.

The door 16 internally comprises supporting elements 18 for the mother bag. The elements 18 are connected to a load cell for detecting the weight of the mother bag.

The door 16 also has, on the side opposite to that where the rotation pin 17 is provided, one or more pins 19, able to engage with corresponding attachment elements 20.

The clamping elements 20 are connected solidly to each other, inside the box-like body 11, and at least one of them has a gripping portion 21.

On one wall 23 of the seating 15 near which the pin 17 is positioned, one or more pressers 22 to semi-automatically open the door 16 are made.

The pressers 22 can be, for example, pins that protrude from the wall 23 and are associated with elastic thrust means which tend to keep them in a protruding position.

The pressers 22 could also be made on the internal surface of the door 16 and could abut, for example, against a portion of wall delimiting the seating 15.

When the door 16 is closed, the pressers 22 are pressed by the internal surface 24 of the door 16 against the action of the elastic means and the door 16 is held in the closed position by the pins 19 engaging in the attachment elements 20.

When the attachment elements 20 are disengaged from the pins 19, for example by lifting the attachment elements 20 by means of the gripping portion 21, the door 16 opens automatically thanks to the thrust of the pressers 22, which quickly protrude from the wall 23 and cause the door 16 to rotate open. The attachment elements 20 could also be raised by means of a suitable servo mechanism commanded by the control unit of the apparatus 10.

Inside the seating 15 where the mother bag will be positioned, a presser plate 25 is also provided, which advantageously has a flat pressing surface.

The flatness of the plate 25 therefore allows a uniform and controlled pressing of the mother bag.

The flatness and uniformity of the surface of the presser plate 25 therefore allows maximum accuracy and efficiency in managing the streams of hematic fluid generated as a result of the pressing, and flowing from the mother bag to the satellite bags.

The plate 25 can be made, for example, by means of a glass plate of suitable thickness.

The presser plate 25 will be associated with drive means which allow it to translate bi-directionally inside the seating 15, so as to compress the mother bag or move away from it.

The plate 25 is substantially parallel to a bottom wall 26 of the seating 15, in which optical elements 27 can be positioned to continuously detect and monitor the buffy coat level or central layer of platelets and white blood cells inside the mother bag.

The optical elements 27 could be, for example, LEDs or suchlike.

If the plate 25 is made in the form of a transparent glass plate, the optical elements 27 could be disposed on a support of the plate 25 positioned behind it.

Alternatively, the plate 25 could comprise a window that leaves the optical elements 27 the possibility of detecting the level of the central layer of the mother bag.

In this variant embodiment of the apparatus 10 two columns of twelve optical elements 27 are provided.

Naturally, the number of optical elements 27 could be different from the one shown, and a single column of optical elements 27 could also be provided.

In any case, advantageously, by means of the optical elements 27 it is possible to perform a precise reading of the buffy coat level, and the undesirable overlapping of the light beams used for reading is advantageously prevented, which could cause inaccuracies in determining said level.

The number and disposition shown of the optical elements 27 also allow to have greater precision with the hematocrit, that is, to estimate more accurately how many red blood cells present in the lower layer of the bag to associate with the central layer of platelets and white blood cells, or buffy coat.

For example, it can be supposed that the group of optical elements 27B situated in the central zone of the two columns represent one of the possible ranges inside which the central layer of platelets and white blood cells or buffy coat is positioned. In this case, the selected extraction program provides an average hematocrit value in the resulting buffy coat or intermediate layer.

If the intermediate layer or buffy coat is maintained during the separation process in correspondence with the optical elements 27 positioned above said optical elements 27B, the hematocrit will be higher than said average value, hence a greater percentage of red blood cells will remain inside the mother bag; whereas if the intermediate layer or buffy coat is maintained during the separation process in correspondence with the optical elements 27 positioned under the optical elements 27B, the hematocrit will be lower than said average value.

At the side of the box-like body 11 of the apparatus 10 another optional presser plate 28 is provided, which can be translated bi-directionally toward and away from the box-like body 11 of the apparatus 10 by means of suitable drive means situated inside the box-like body 11.

In the vicinity of the plate 28 a housing and support seating is provided, not visible in the drawings, which can be used for a mother bag or for one of the satellite bags.

Around the box-like body 11 one or more supports or positioning seatings for satellite bags can be positioned, see for example the seatings 29 and 30.

The support seatings 29 and 30 will be connected to detectors to detect the weight of the bags.

The apparatus 10 also comprises, inside the box-like body 11, one or more RFID-type communication modules 31 connected to a control unit located inside the box-like body 11.

The modules 31 are shown by dotted lines in fig. 1 and can comprise RFID type antennas, for example of a quadrangular or similar shape, of variable size and associated with corresponding control boards.

The modules 31 are preferably positioned close to each of the seatings provided for the mother bag and the satellite bags, for example near the seating 15, the seating 29, the upper part of the box-like body 11, where a bag can be rested, and close to the lateral plate 28.

The RFID-type communication modules 31 have the advantage that they are able to automatically read and recognize the data associated with each of the bags, both the mother bag and the satellite bags, which will be provided with a suitable label, with microchips or suchlike. In current apparatuses, this recognition is carried out manually by means of a kind of pistol for reading the bar codes of the bags.

In essence, therefore, by means of the modules 31, it is advantageously possible to automatically read each bag, mother or satellite, that passes in the apparatus 10.

The modules 31, in addition, or in alternative to the above, can advantageously also allow to write data on the bags.

The communication modules 31 also allow, in addition or in alternative to the above, a cross-check of the bags being processed in the apparatus 10 with the database of a transfusion center and/or the automatic launch and activation of predefined procedures and processes even without intervention of an operator.

The communication modules 31 also allow, in addition or in alternative to the above, write from the blood components data resulting from the extraction processes.

The modules 31 also allow complete traceability of the bags obtained from the extraction process performed by the apparatus 10, and therefore the mother bag with hematic fluid having a desired quantity of platelets and white blood cells and satellite bags containing red cells and plasma.

As can be seen, the present apparatus comprises, by way of example, five multifunction clamps 14a-14e, so in essence, up to five satellite bags could be used to collect the blood components extracted from the mother bag.

Each of the multifunction clamps 14a-14e, see for example, any one whatsoever of clamps 14a-14e shown in figs 3, 4, 5 and 6, comprises a support body 33 having internally a mobile module 34 associated with a hot electrode 35 able to translate upward or downward.

By the term "hot electrode" we mean the electrode that receives the radiofrequency signal RF, as will be seen hereafter.

The hot electrode 35 can then be translated upward or downward by means of the mobile module 34, which is driven by means of a corresponding command device 41, for example an electromagnet.

It is possible to provide that, instead of the electromagnet, the command device 41 comprises, for example, a motor for proportional control. It is also possible to provide that some clamps 14a-14e are provided with electromagnets and others with motors.

The hot electrode 35 protrudes from a support plate 37 provided with a threaded crown 38 able to engage with a ring nut 39 associated with a lid 40 of the multifunction clamp 14a-14e.

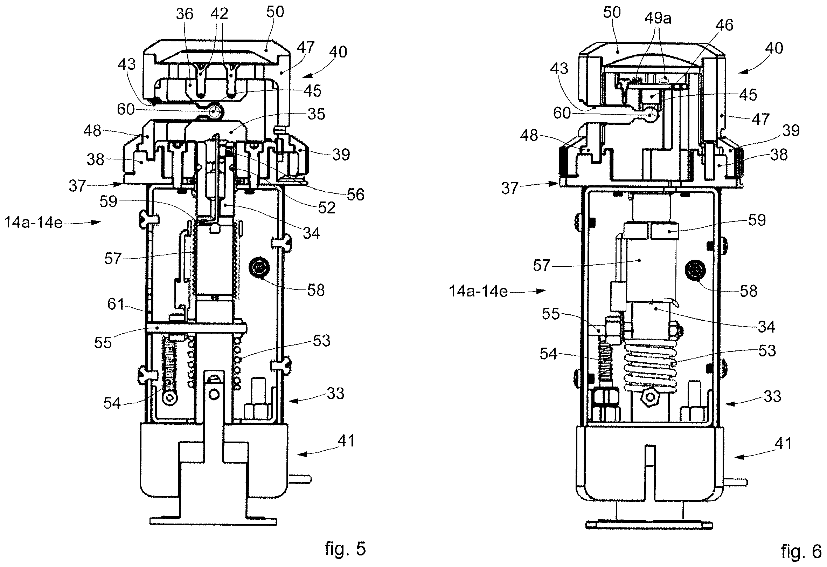

As can be seen from the sections of figs. 5 and 6, the ring nut 39 can translate with respect to the body 47 of the lid 40 and, when screwing is completed, rests on an abutment element 48, for example an annular element.

In the lid 40, which can be screwed and unscrewed to/from the support body 33 of the multifunction clamp 14a-14e, a cold electrode 36 is housed.

By the term "cold electrode" we mean the electrode that is grounded.

The cold electrode 36 is attached to the lid 40 by removable attachment means 42, such as screws or suchlike.

Advantageously therefore, the removal of the lid 40 of the multifunction clamp 14a-14e also allows to remove the cold electrode 36, so as to guarantee better and easier access to all parts of the multifunction clamp and better cleaning thereof.

The multifunction clamp 14a-14e thus conceived also has improved electrical operating stability.

The lid 40 comprises a seating 43 for inserting the tube 60.

On the support plate 37 and at the sides of the hot electrode 35 two uprights 44 are made, which comprise grooves 45 to retain the tube 60.

The grooves 45 are positioned substantially at the height of the hot electrode 35.

Sensors 46 to detect the presence of the tube 60, for example optical or mechanical sensors or others, can be provided in the uprights 44, for example in correspondence with the grooves 45.

On top of each of the uprights 44 one or more optical elements 49a or 49b are positioned, for example, luminous elements such as LEDs or suchlike. The optical element 49a is selected in a first color, for example red, while the optical element 49b is selected in a second color, different from the first color, for example green.

The first optical element 49a is able to indicate that in that particular multifunction clamp 14a-14e the tube 60 through which the hematic fluid passes has to be inserted, according to the extraction program selected. If, for example, the optical element 49a is switched on, it means that according to the extraction program selected, the tube 60 has to be inserted in that multifunction clamp.

The second optical element 49b lights up instead when the tube 60 has been inserted correctly. Consequently, the first optical element 49a will be switched off.

Therefore, in essence, according to the above example, the multifunction clamps selected by the apparatus 10 for a particular extraction program will light up red and, once the tube 60 has been correctly inserted into the selected multifunction clamps, the latter will light up green.

In order to make the lighting of the multifunction clamps 14a-14e more visible, both in the first color and in the second color, a diffuser 50 is provided on the upper part of the body 47 of the lid 40, made of a substantially transparent or slightly opaque material.

It is possible to position, on the diffuser 50, a label 51 to recognize the multifunction clamp 14a-14e.

At least one sealing element 52, for example an O-ring or suchlike, see fig. 5, is associated with the mobile module 34 which carries the hot electrode 35 so as to prevent any possible infiltrations of liquids, in particular from the upper part of the multifunction clamp 14a-14e.

The mobile module 34 is associated with preloading elastic means 53 housed inside the support body 33 of the multifunction clamp. Elastic means 54 can also be provided inside the support body 33, to restore the position of the mobile module 34, and are associated with an end-of-travel element 55.

The end-of-travel element, in an initial position of the mobile module 34, is able to abut on one end of a slit 61 made in the support body 33 of the clamp 14a-14e.

The hot electrode 35 is held in position on the mobile module 34 by means of a grub screw 56 or other attachment system such as a screw or suchlike.

As mentioned above, the hot electrode 35 is electrically powered by a suitable power supply device, for example a radio frequency coil 57.

The radio frequency coil 57 is connected to a corresponding power connector 58.

The radio frequency coil 57 is also able to cooperate with a position calibration ring 59.

As mentioned above, by means of the monitor 12 it is possible to select a program to extract blood components from a mother bag comprising centrifuged whole blood.

Depending on the program chosen and the positioning of the satellite bags on the apparatus 10, the control unit of the apparatus 10 will switch on the optical elements 49a of the multifunction clamps in which the tubes to remove the red blood cells or plasma from the mother bag, positioned in the seating 15, must be inserted.

For example, it is possible to provide that a satellite bag for collecting red blood cells is positioned on the support seating 29. The seating 29 is connected to a weight detector or, alternatively, the red blood cell collection bag can be housed on an external mixing and weighing device, not shown. In this case, the tube 60 for removing the red blood cells from the lower part of the mother bag will be positioned inside the multifunction clamp 14e. By choosing this type of removal, the apparatus 10 will command the optical element 49a associated with the multifunction clamp 14e to switch on, indicating that the tube 60 through which the hematic fluid flows must be inserted in this clamp 14e, in this case the fluid with greatest density and rich in red blood cells. When the tube 60 has been inserted correctly in the seating 43 of the lid 40 and between the grooves 45 of the multifunction clamp 14e, the sensors 46 will signal to the control unit of the apparatus 10 that the tube 60 is in position. At this point, the control unit will command the optical element 49a, for example red, to switch off, and will command the optical element 49b, for example green, to switch on, in order to signal that the tube 60 has been inserted and positioned correctly.

Another tube for removing plasma from the mother bag can be inserted, for example, in the multifunction clamp 14d, the functioning of which is analogous to that described above for the multifunction clamp 14e.

While the plasma and red blood cells are being removed from the mother bag, the plate 25 suitably presses the mother bag and we have a substantially alternate delivery to the satellite bag for collecting plasma and to the satellite bag for collecting red blood cells, so as to maintain a more or less constant, or slightly variable, level of the intermediate layer of platelets and white blood cells, or buffy coat. As we said, this level is promptly detected by the columns of optical elements 27 and, depending on the program selected, will be kept around a certain group of optical elements 27 as mentioned above. The door 16 substantially serves as a contrast plate when the plate 25 presses the mother bag of whole blood.

When the desired quantity of platelets and white blood cells is present in the mother bag, and the desired quantity of red cells and plasma is collected in the satellite bags, the apparatus 10 commands the welding of the tubes 60, in this example of the tubes 60 which will be housed in the multifunction clamps 14e and 14d.

The radiofrequency welding of each tube 60, generally made of plastic and soft material, is effected by suitably moving the hot electrode 35 close to the cold electrode 36 and then by powering the hot electrode 35 through the radiofrequency coil 57.

During the welding, the hot electrode 35 and the cold electrode 36 are extremely close to one another and between them the tube 60 compressed in the welding zone is positioned.

The welding lasts a few seconds and it can be provided, by means of the control unit of the apparatus 10, that the welding is adapted automatically to the characteristics of the tube to be welded, for example by following a given welding profile.

It can be provided that after welding is completed, another luminous signal is emitted by the optical elements 49a and 49b, for example by providing them to be switched off.

Providing the optical elements 49a and 49b on the top of the uprights 44 guarantees better visibility thereof and prevents the risk of contact of the same with the tube in which hematic fluid flows.

As an alternative to what is shown and described, it is also possible to provide only one optical element 49a or 49b which is able to vary, through a command by the control unit of the apparatus, from at least one first coloring that indicates the need to insert the tube 60 in the multifunction clamp 14a-14e, according to the extraction program selected, to at least one second coloring to indicate the correct insertion of the tube 60 into the multifunction clamp 14a-14e.

It is clear that modifications and/or additions of parts can be made to the extractor apparatus for blood components as described heretofore, without departing from the field and scope of the present invention.

It is also clear that, although the present invention has been described with reference to some specific examples, a person of skill in the art shall certainly be able to achieve many other equivalent forms of extractor apparatus for blood components, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. Extractor apparatus for blood components, suitable for use in the field of medical blood transfusion for transferring at least one blood component between at least one mother bag containing centrifuged whole blood and at least one satellite bag, **characterized in that** it comprises at least one housing seating (15) of the mother bag and one or more housing seatings (28, 29, 30) of one or more satellite bags, **and in that** it comprises one or more communication modules (31) of the RFID type installable in correspondence to said housing seatings (15, 28 29, 30) of the mother bag and/or the satellite bags and configured to perform one or more between an automatic recognition of the mother bag and/or the satellite bags, to write the data relating to the blood components resulting from the extraction process, to perform cross-checks with transfusion center database, and to allow an automatic processes activation even without operator intervention.

2. Extractor apparatus as in claim 1, **characterized in that** it comprises a plurality of multifunction clamps (14a, 14b, 14c, 14d, 14e) able to hold, control the flow of hematic fluid and weld at least one tube (60) configured to connect the mother bag to at least one of the satellite bags.

3. Extractor apparatus as in claim 1 or 2, **characterized in that** said housing seating (15) of the mother bag is provided with means (25) for the controlled pressing of the mother bag.

4. Extractor apparatus as in any claim 2 or 3, **characterized in that** said multifunction clamps (14a, 14b, 14c, 14d, 14e) are provided with one or more optical elements (49a, 49b) able to indicate in a programmed way the insertion of the tube (60) and at least a situation of correct holding of the tube (60).

5. Extractor apparatus as in any claim 2 to 4, **characterized in that** said multifunction clamps (14a, 14b, 14c, 14d, 14e) comprise at least a sealing element (52) against possible infiltrations of fluids.

6. Extractor apparatus as in any claim 2 to 5, **characterized in that** said multifunction clamps (14a, 14b, 14c, 14d, 14e) comprise at least a support body (33) with which a removable lid (40) is associated.

7. Extractor apparatus as in claim 6, **characterized in that** said multifunction clamps (14a, 14b, 14c, 14d, 14e) comprise at least a hot welding electrode (35) and at least a cold welding electrode (36), at least one of either said electrodes (35, 36) being associated with said lid (40).

8. Extractor apparatus as in claims 4 and 6, **characterized in that** said lid (40) is provided with at least a diffuser (50) able to spread the light signals coming from said optical elements (49a, 49b) situated in a zone of the multifunction clamp (14a, 14b, 14c, 14d, 14e) below said lid (40).

9. Extractor apparatus as in claim 7, **characterized in that** said hot welding electrode (35) is associated with a mobile module (34) provided with said sealing element (52).

10. Extractor apparatus as in any claim 2 to 9, **characterized in that** said multifunction clamps (14a, 14b, 14c, 14d, 14e) comprise at least one pair of uprights (47) comprising grooves (45) to hold the tube (60) and one or more sensors (46) to detect the presence of the tube (60).

11. Extractor apparatus as in any claim hereinbefore, **characterized in that** it comprises a door (16) to close the housing seating (15) of the mother bag provided with semi-automatic opening means (19, 20, 22).

12. Extractor apparatus as in claim 3, **characterized in that** said pressing means comprise at least a plate (25) that has a substantially flat and uniform pressing surface.

13. Extractor apparatus as in any claim hereinbefore, **characterized in that** the housing seating (15) of the mother bag comprises at least a column of optical elements (27) of which at least one group is provided with one or more optical elements (27B) configured to detect precisely a range of the level of the layer of platelets and white blood cells to keep inside the mother bag.
